# EUROPÄISCHE PATENTSCHRIFT

(11) **EP 1 286 655 B1**
(45) Veröffentlichungstag und Bekanntmachung des Hinweises auf die Patenterteilung: **13.09.2006**
(21) Anmeldenummer: 01951517.0
(22) Anmeldetag: 23.05.2001
(51) Int. Cl.: A61Q 5/12, A61K 8/37, A61K 8/40

(54) **TOPISCHE ZUSAMMENSETZUNG, ENTHALTEND MINDESTENS EIN ARYLOXIM, UND VERFAHREN ZU IHRER HERSTELLUNG**
TOPICAL COMPOSITION CONTAINING AT LEAST ONE ARYL OXIME, AND METHOD FOR THE PREPARATION THEREOF
COMPOSITION TOPIQUE COMPRENANT AU MOINS UNE ARYLOXIME, ET SON PROCEDE DE PREPARATION

(30) Priorität: 24.05.2000 DE 10025558
(43) Veröffentlichungstag der Anmeldung: 05.03.2003
(73) Patentinhaber: Merck Patent GmbH, 64293 Darmstadt (DE)
(72) Erfinder: BÜNGER, Joachim, 64853 Otzberg-Hering (DE); ZUR LAGE, Jutta, 64289 Darmstadt (DE); SCHWARZ, Michael, 64331 Weiterstadt (DE)
(74) Vertreter: Grünecker, Kinkeldey, Stockmair & Schwanhäusser Anwaltssozietät
(86) Internationale Anmeldenummer: PCT/EP2001/005953
(87) Internationale Veröffentlichungsnummer: WO 2001/089469

(56) Entgegenhaltungen:
- EP-A- 1 066 821
- WO-A-95/01157
- DE-A- 4 116 123
- DE-A- 19 722 405
- FR-A- 2 788 694
- US-A- 4 816 487
- US-A- 5 079 265

## Beschreibung

Die vorliegende Erfindung betrifft eine topische Zusammensetzung, enthaltend mindestens ein Aryloxim, die über einen längeren Zeitraum stabil bleibt und eine gute Penetration in die Haut gewährleistet. Weiterhin betrifft die vorliegende Erfindung ein Verfahren zur Herstellung dieser topischen Zusammensetzung.

Bei vielen Krankheiten sind Entzündungen als Symptome zu beobachten, die entweder ursächlich oder infolge krankhafter Veränderungen sekundär in Erscheinung treten. Sie können ebenfalls durch chemische oder physikalische Noxen von außen hervorgerufen werden. Eine Entzündung ist ein multifunktioneller Vorgang von unterschiedlichen morphologischen und funktionellen Faktoren. Diese Faktoren betreffen dabei sowohl Störungen im zellulären Bereich, bei der Blutzirkulation, entzündungsbedingte Trans- und Exsudation, Infiltration und Proliferation. Im Gefolge dieser Störungen können weitere Veränderungen auftreten, so daß u.a. Spongiose, Akanthose oder Parakeratose auftreten.

Bei Auslösung, dem Ablauf und der Steuerung vieler dieser Vorgänge sind Mediatorsysteme beteiligt. So sind die von sensibilisierten T-Lymphozyten abgegebenen Lymphokinine maßgeblich mit einer großen Zahl biologischer Wirkungen an der zellulären Immunantwort beteiligt (Schöpf, E., Korting, G.W. (Herausgeber) Dermatologie u. Praxis, Bd. 1, Thieme: Stuttgart, New York (1980)). Weiterhin sind in diesem Zusammenhang die Wirkung von Kininen, aktivierten Komplementfaktoren, lysosomalen Enzymen, zyklischen Nukleotiden und verschiedenen epidermalen Faktoren bekannt. Eine besondere Rolle spielen die Prostaglandine und Leukotriene. Als Beispiel einer Prostaglandinwirkung ist eine chemotaktische Wirkung auf Leukotriene bekannt, die zeitlich nach den Kininen die Gefäßpermeabilität vermindert. Dagegen wirken Leukotriene chemotaktisch auf Granulozyten und beeinflussen die Kontraktibilität und Permeabilität von Gefäßen.

Ein UV-B Erythem wird, abgesehen von der Histaminfreisetzung, durch die Arachidonsäurekaskade vermittelt, wobei eine gesteigerte Cyclooxygenase-vermittelte Prostaglandinsynthese, insbesondere von PGE₂ und PGF₂, vorliegt. Der Lipoxygenaseweg über 5-HPETE und LTA4 führt zu wesentlichen Elementen der Entzündung, wie zelluläre Infiltration des entzündeten Gewebes und Ödembildung (Übersicht bei: Gallin, J., Goldstein, I.M., Snyderman R. (Herausgeber), Inflamation. Basic principles and clinical correlates, New York, Raven Press (1988)).

Zur Behandlung von Entzündungen sind verschiedene Wirkstoffe bekannt. Die größte Bedeutung in der Behandlung der vorstehend genannten Mechanismen, die zu unterschiedlichen Hauterkrankungen führen, haben Corticosteroide. Schwache bis mittelstarke Corticosteroide, meist nicht fluorierte Derivate des Hydrocortison, werden vorwiegend zur Therapie entzündlicher, allergischer und pruriginöser Hauterkrankungen eingesetzt. Allerdings treten bei der Behandlung mit Corticosteroiden in Abhängigkeit von dem angewandten Wirkstoff, der Art und Dauer der Behandlung unerwünschte Nebenwirkungen auf, die unbedingt bei der Anwendung dieser Substanzen beachtet und berücksichtigt werden müssen (Übersicht: Symposium in Topical Corticosteroids. In: Drugs Bd. 36, 5 (1988)). Aus diesen Gründen werden vorzugsweise nichtsteroidale entzündungshemmende Wirkstoffe eingesetzt, wobei von den bisher bekannten Substanzen die therapeutische Effektivität allerdings sehr begrenzt ist und meist unter der von Hydrocortison liegt. Das betrifft Wirkstoffe, wie Salicylsäure, Acetylsalicylsäure, Bufexamac, Bendazac, Phenylbutazon, Oxyphenbutazon, Diflumidon, Indometacin und teilweise auch Antihistaminika (Gloor, M.: Pharmakologie dermatologischer Extema. Springer Verlag Berlin Heidelberg New York, (1982)).

In der EP-A-0149 242 werden als Wirkstoffe u.a. zur Behandlung von Hauterkrankungen 1-(2-Hydroxyaryl)-alkan-1-on-oxime vorgeschlagen. Die Wirkstoffe können dabei oral, perlingual, rektal, parenteral, intravenös oder percutan sowie als Aerosol angewendet werden. Als pharmazeutische Zubereitung werden u.a. Suspensionen und Emulsionen, Pasten, Salben, Gele, Cremes und Lotionen genannt. In einem Beispiel wurde 2-Hydroxy-5-methyl-laurophenon-oxim intraperitonal appliziert, und die Hemmung des Carrageeninödems der Rattenpfote wurde untersucht.

Neben den Eigenschaften des Wirkstoffes und des morphologischen und funktionellen Zustandes der zu behandelnden Hautareale sind es vor allem die Eigenschaften des benutzten Vehikels, von denen die therapeutische Effektivität eines topisch applizierten Arzneimittels abhängig ist. Insgesamt wird ein ausreichendes, optimales Konzentrations-Zeit-Profil des Wirkstoffs in der erkrankten Hautschicht angestrebt.

Die EP-B-389 773 offenbart ein Verfahren zur Herstellung einer galenischen Zubereitung mit optimaler Bioverfügbarkeit des Wirkstoffes 2-Hydroxy-5-methyl-laurophenon-oxim. Dabei werden Gallensäuren als Resorptionsmittel verwendet. Als Gallensäuren können beispielsweise Desoxy- oder Dehydrocholsäure oder Gemische dieser in Form ihrer Salze verwendet werden. Die Formulierung kann in Form von Lösungen, Suspensionen, Kapseln, Granulaten, Tabletten oder Dragees erfolgen.

In der DE-A-41 16 123 werden topische Zusammensetzungen auf der Basis von 2-Hydroxy-5-methyl-laurophenon-oxim offenbart. Als Anwendungsgebiete werden die pharmazeutische Industrie, die Human- und Veterinärmedizin sowie die Kosmetik genannt. Neben dem Wirkstoff enthalten die Formulierungen weiterhin normalerweise in der Haut vorkommende hydratisierende Substanzen und/oder Moisturizer und/oder Pentetrationspromotoren und andere Stoffe. Die Wirkstoffkonzentration beträgt üblicherweise 0,1 bis 50%, und die vorstehend genannten zusätzlichen Substanzen liegen in einer Menge von 0,1 bis 40% vor. Als bevorzugte hydratisierende Substanz und/oder Moisturizer wird Harnstoff eingesetzt. Bevorzugte Pentetrationspromotoren beinhalten Propylenglykol und Gallensäure. Die topische Formulierung liegt in Form von W/O-Emulsionen, Hydrogelen oder Mischgelen in Form von wasserfreien und/oder wasserhaltigen und lipohilen Salben, wasserhaltigen lipophilen Salben oder nichtionischen Cremes, Pasten, Schüttelmixturen, Lotionen und Emulsionen vor. Die Einarbeitung von Harnstoff konnte die Penetration des Wirkstoffes in die verschiedenen Schichten der menschlichen Haut sowie die Liberation des Wirkstoffs aus der topischen Formulierung erhöhen. Der Zusatz von Propylenglykol und Natriumdesoxycholat als Penetrationspromotoren konnte diesen Effekt weiter verbessern.

Ein großes Problem bei der topischen Anwendung der vorstehend genannten Verbindungen stellt deren geringe Löslichkeit in kosmetischen und dermatologischen Formulierungen und deren Neigung zum Auskristallisieren dar. Insbesondere ist der Wirkstoff unter Wärmezufuhr in Emulsionssystemen löslich, jedoch wird nach dem Abkühlen sowohl in O/W- als auch in W/O-Emulsionen während der Lagerung ein Auskristallisieren dieses Wirkstoffes beobachtet. Selbst wenn der Wirkstoff innerhalb der Emulsionssysteme gelöst bleibt, kann es trotzdem nach Auftragen der Formulierung zu Auskristallisierungen in den Hautschichten kommen.

Zahlreiche Untersuchungen wurden durchgeführt, um optimale Wirkstoffzusammensetzungen zu erhalten, bei denen es zu einer Verbesserung der Löslichkeit und einem Verhindern des Auskristallisierens kommt. Obwohl Aryloxime, insbesondere 2-Hydroxy-5-methyl-laurophenon-oxim, in verschiedenen Lösungsmitteln, wie Isopropanol, Aceton, Chloroform, 2-Phenylethanol und Triton-XIOO, gut löslich sind, eignen sich diese Träger nicht oder nur bedingt für eine Verwendung für kosmetische und dermatologische Formulierungen. Deshalb müssen die Träger nichttoxisch, nichtkarzinogen und hautverträglich sein und sollten weiterhin duftneutral sein. Weiterhin sollten diese Träger, z.B. Lösungsmittel oder Emulgatoren, mit dem Hauptanteil der topischen Zusammensetzung, d.h. einer wäßrigen Phase oder einer Ölphase, kompatibel sein und keine getrennte Phase bilden oder zur Ausfällung des Wirkstoffes führen.

Bei Untersuchungen zur Löslichkeit von insbesondere 2-Hydroxy-5-methyl-laurophenon-oxim konnten Lösungen hergestellt werden, die bis zu vier Tage bei Raumtemperatur unter Ausschluß von UV-Licht stabil waren. Bei diesen Lösungen handelte es sich um ethanolische Wirkstofflösungen, die weitere Bestandteile, wie 0,8/1,0 und 2,0% PEG600 bzw. PEG6000 enthielten. Der Wirkstoff lag in diesen Lösungen in einer Menge von 10 Gew.% vor. Bei einer Erhöhung der Wirkstoffmenge kam es zu Auskristallisierungen. Diese Lösungen können in Wasser eingearbeitet werden, um topische Formulierungen herzustellen. Bei einem Anteil der Wirkstofflösung in der Formulierung von 10 bis 20 Gew.% kam es jedoch vereinzelt zum Auskristallisieren von 2-Hydroxy-5-methyl-lauro-phenon-oxim.

Weiterhin kommt es üblicherweise zu einer unerwünschten Verfärbung, z.B. Gelbfär bung, wenn der Wirkstoff in eine der vorstehend beschriebenen Formulierungen ge bracht wird.

WO 95/01157 A1 beschreibt eine photoprotektive Zusammensetzung enthaltend Glycerylstearat und Hydroxyphenyloxim in einem pharmazeutisch akzeptabeln Träger.

EP-A-1066821 beschreibt topische kosmetische Mittel, die Benzaldoxime mit mindestens einer aromatischen Hydroxy- oder Alkoxygruppe enthalten, für kosmetische oder dermatologische Anwendungen, insbesondere als Hautaufhellungsmittel.

FR-A-9900882 betrifft eine Zusammensetzung zur topischen Applikation, welche Derivate von Phenylhydroxim enthält.

Aus der DE-A-19722405 ist bekannt, dass als Dispergierhilfen Reaktionsprodukte, wie Ester oder Ether, aus langkettigen Carbonsäuren oder langkettigen Alkoholen einerseits mit Hydroxylgruppen aufweisenden oder bildenden Verbindungen wie Glycerin, Ethylenoxid, Propylenoxid, Milchsäure oder Weinsäure andererseits eingesetzt werden können.

Es ist deshalb die Aufgabe der vorliegenden Erfindung, eine topische Zusammensetzung, enthaltend mindestens ein Aryloxim, zur Verfügung zu stellen, die eine verbesserte Löslichkeit sowie eine Stabilisierung des Wirkstoffes aufweist und ein Auskristallisieren des Wirkstoffes verhindert, so daß es zu einer Erhöhung der Bioverfügbarkeit des Aryloxims kommt. Gleichzeitig soll die Zusammensetzung ein Vermeiden von Verfärbungen, eine gute Penetration zum gewünschten Wirkungsort sowie eine gute Liberation des Wirkstoffes neben einer guten Hautverträglichkeit gewährleisten.

Diese Aufgabe wird gelöst durch eine topische Zusammensetzung, enthaltend
(a) mindestens ein Aryloxim der Formel (I) und
(b) mindestens einen Emulgator, 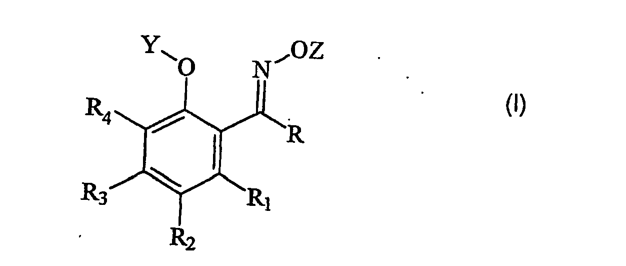 worin bedeuten:
   - Y, Z: unabhängig voneinander H, C₁₋₁₈-Alkyl, C₂₋₁₈-Alkenyl, C₂₋₁₈₋Carboxyalkyl, C₃₋₁₈-Carboxyalkenyl oder C₂₋₁₈-Alkanoyl;
   - R: C₁₋₁₈-Alkyl, C₂₋₁₈-Alkenyl, C₃₋₈-Cycloalkyl, Aryl, Aralkyl, Heteroaryl, Heteroaralkyl oder kondensierte Systeme;
   - R₁, R₂, R₃ und R₄: unabhängig voneinander H, C₁₋₁₂-Alkyl, C₂₋₁₂-Alkenyl, C₁₋₁₂-Alkoxy, C₃₋₈-Cycloalkoxy, Aryl, Aryloxy, Aralkyl, Heteroaryl, Heteroaralkyl, Carboxy, Hydroxy, Chlor, Dialkylamin oder Sulfonyl,
dadurch gekennzeichnet, daß die Komponente (b) ausgewählt wird aus der Gruppe, bestehend aus mindestens einem Ester, dessen Carbonsäurerest sich von C₅- bis C₁₆₋Säuren ableitet und dessen Hydroxylrest sich von Monomeren, Dimeren oder Trimeren der Milchsäure oder eines ihrer Salze oder einem Polyglycerin aus 2 bis 10 Molekülen Glycerin ableitet, wobei pro mol Polyglycerin 1 bis 3 mol Carbonsäure vorliegen.

Die vorliegende Erfindung betrifft ebenfalls ein Verfahren zur Herstellung der vorstehend genannten topischen Zusammensetzung, umfassend die Schritte
- Herstellung einer Phase A durch Mischen der Komponenten (a) und (b) und
- Einarbeitung der erhaltenen Phase A in eine Phase B, enthaltend einen Träger.
   Die erfindungsgemäße topische Zusammensetzung ist zur Prophylaxe und/oder Behandlung von Hauterkrankungen und/oder Entzündungsreaktionen der Haut geeignet. Weiterhin kann die erfindungsgemäße topische Zusammensetzung zur kosmetischen Pflege der Haut verwendet werden.
   Es wurde überraschend gefunden, daß die Aryloxime am Auskristallisieren gehindert werden, wenn sie zusammen mit bestimmten Emulgatoren vorliegen, die diese Verbindungen in eine flüssig-kristalline (LC)-Phase integrieren und in diesem Zustand stabilisieren können. Dabei kommt es zur sogenannten liposomalen Verkapselung, die dauerhaft jegliche Kristallisationsphänomene unterdrücken kann. Entscheidend für die Stabilität dieser vesikelförmigen Membranen ist im wesentlichen die Kompatibilität zwischen der Struktur des zu verkapselnden Wirkstoffes und des entsprechenden Trägersystems. Dabei ist die vollständige Löslichkeit in dem vesikelbildenden Emulgator eine Voraussetzung. Die Stabilisierung verhindert ebenfalls eine Verfärbung der Zusammensetzung.
   Abbildung 1 zeigt die Ergebnisse der in Beispiel 7 durchgeführten visuellen Begutachtung zur Bestimmung der Erythemstärke der Prüfareale 1 und 2, bei denen eine Vorbehandlung mit den jeweiligen Prüfpräparaten stattfand.
   Abbildung 2 zeigt die Ergebnisse der in Beispiel 7 durchgeführten visuellen Begutachtung zur Bestimmung der Erythemstärke der Prüfareale 3 und 4, bei denen eine Nachbehandlung mit den jeweiligen Prüfpräparaten stattfand.
   Die erfindungsgemäße topische Zusammensetzung enthält als Komponente (a) mindestens ein Aryloxim der Formel (I) 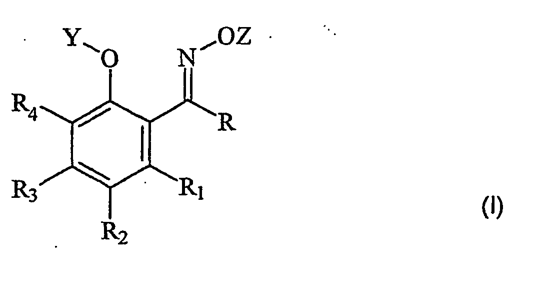
worin bedeuten:
- Y, Z: unabhängig voneinander H, C₁₋₁₈-Alkyl, C₂₋₁₈-Alkenyl, C₂₋₁₈₋Carboxyalkyl, C₃₋₁₈-Carboxyalkenyl oder C₂₋₁₈-Alkanoyl;
- R: C₁₋₁₈-Alkyl, C₂₋₁₈-Alkenyl, C₃₋₈-Cycloalkyl, Aryl, Aralkyl, Heteroaryl, Heteroaralkyl oder kondensierte Systeme;
- R₁, R₂, R₃ und R₄: unabhängig voneinander H, C₁₋₁₂-Alkyl, C₂₋₁₂-Alkenyl, C₁₋₁₂-Alkoxy, C₃₋₈-Cycloalkoxy, Aryl, Aryloxy, Aralkyl, Heteroaryl, Heteroaralkyl, Carboxy, Hydroxy, Chlor, Dialkylamin oder Sulfonyl.

Alkyl, Alkenyl, Carboxyalkyl, Carboxyalkenyl, Alkanoyl, Cycloalkyl, Alkoxy, Aryl, Aryloxy und Aralkyl können unsubstituiert oder substituiert sein. Als Substituenten dieser Gruppen kommen vorzugsweise Alkyl, Alkoxy, Alkenyl, Aryl, Aryloxy, Aralkyl, Heteroaryl, Heteroaralkyl, Hydroxy, Carboxy, Carboxyalkyl, Dialkylamin, Sulfonyl und Kombinationen davon in Frage.

Alkyl bedeutet jeweils geradkettiges oder verzweigtes Alkyl und bedeutet daher bevorzugt Methyl, Ethyl, Propyl, Isopropyl, Butyl, Isobutyl, t-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl und Octadecyl.

Alkenyl bedeutet, daß in dem spezifizierten Alkylen eine oder mehrere Doppelbindungen vorhanden sein können.

Aryl steht für einen aromatischen C₆₋₂₀-Kohlenwasserstoffrest und bedeutet vorzugsweise Phenyl.

Aralkyl bedeutet eine mit Aryl substituierte Alkylgruppe und hat vorzugsweise die Bedeutung von Benzyl oder Phenethyl.

Cycloalkyl bedeutet eine cyclische Alkylgruppe und ist vorzugsweise Cyclopropyl, Cyclobutyl, Cyclopentyl, Cyclohexyl, Cycloheptyl oder Cyclooctyl.

Heteroaryl steht für einen aromatischen Ring mit Heteroatomen, vorzugsweise für einen stickstoffhaltigen Ring, wie Pyridinyl oder Pyrimidinyl.

Heteroaralkyl bedeutet eine mit Heteroaryl substituierte Alkylgruppe und ist vorzugsweise Pyridinylmethyl und Pyrimidinylmethyl.

Als kondensierte Systeme kommen vorzugsweise die Reste Naphthyl, Benzofuryl, Chinolinyl, Indolyl oder Cinnolinyl in Betracht.

Dialkylamin steht für NR₅R₆, wobei R₅ und R₆ gleich oder unterschiedlich sein können und C₁₋₁₂-Alkyl bedeuten.

Z und Y sind vorzugsweise unabhängig voneinander ein Wasserstoffatom, eine C₁₋₆₋Alkylgruppe, die mindestens einen Substituenten, ausgewählt aus -OH, -COOH, -SO₃H oder NR₅R₆, besitzen kann, eine Alkanoylgruppe, dargestellt durch -C(O)R₇, worin R₇ eine C₁₋₆-Alkylgruppe, die mindestens einen Substituenten, ausgewählt aus -OH, -COOH oder -SO₃H besitzen kann, oder eine CONHR₈-Gruppe, worin R₈ eine C₆₋₂₀₋Arylgruppe bedeutet. Besonders bevorzugt sind Z und Y unabhängig voneinander ein Wasserstoffatom, -(CH₂)₁₋₆COOH, -CH₂CH(OH)CH₂OH, -(CH₂)₁₋₆SO₃H, -(CH₂)₁₋₆NR₅R₆ oder C(O)(CH₂)₁₋₆COOH.

Der Substituent R ist vorzugsweise eine C₁₋₁₂-Alkylgruppe, insbesondere bevorzugt sind C₁₋₅ und C₁₁-Alkylgruppen.

Der Substituent R₁ ist vorzugsweise ein Wasserstoff- oder Chloratom.

Der Substituent R₂ ist vorzugsweise ein Wasserstoff- oder Chloratom oder eine C₁₋₆-Alkylgruppe. Besonders bevorzugt sind ein Wasserstoffatom, ein Chloratom und eine Methylgruppe.

Der Substituent R₃ ist vorzugsweise ein Wasserstoffatom oder eine C₁₋₆-Alkylgruppe, eine C₁₋₆-Alkoxygruppe, eine O-Cyclohexylgruppe oder eine Benzylgruppe.

Der Substituent R₄ ist vorzugsweise ein Wasserstoff- oder Chloratom.

R₁, R₂, R₃ und R₄ können, wenn möglich, vorzugsweise mit -OH, -COOH, -SO₃H oder -NR₅R₆ substituiert sein, um z.B. die Wasserlöslichkeit zu erhöhen.

Bevorzugte Beispiele der Komponente (a) beinhalten:
4-Methyl-2-hydroxy-caprophenon-oxim, 5-Methyl-2-hydroxy-caprophenon-oxim, 5-Methyl-2-hydroxy-caprophenon-(N-phenylcarbamoyl)-oxim, 5-Methyl-2-hydroxy-laurophenon-oxim (2-Hydroxy-5-methyl-laurophenon-oxim), 3-Chlor-2-hydroxy-caprophenon-oxim, 4-Pentoxy-2-hydroxy-acetophenon-oxim, 4-Decyloxy-2-hydroxy-acetophenon-oxim, 4-Benzyloxy-2-hydroxy-acetophenon-oxim, 4-Decyloxy-2-hydroxy-propiophenon-oxim, 4-Butoxy-5-n-hexyl-2-hydroxy-acetophenon-oxim, 4-Pentoxy-2-hydroxy-caprophenon-oxim, 4-Decyloxy-2-hydroxy-caprophenon-oxim, 4-Octyloxy-2-hydroxy-laurophenon-oxim, 4-Cyclohexyl-oxy-2-hydroxy-propiophenon-oxim, 5-Chlor-2-hydroxy-caprophenon-oxim, 3-Chlor-2-hydroxy-laurophenon-oxim, 5-Chlor-2-hydroxy-laurophenon-oxim,4-Butoxy-2-hydroxy-acetophenon-oxim, 4-Dodecyloxy-2-hydroxy-propiophenon-oxim, 4-Hexadecyloxy-2-hydroxy-acetophenon-oxim, 4 Octadecyloxy-2-hydroxy-acetophenon-oxim, 4-Decyloxy-2-hydroxy-laurophenon-oxim, sowie die folgenden Oximderivate von 2-Hydroxy-5-methyl-laurophenon-oxim:

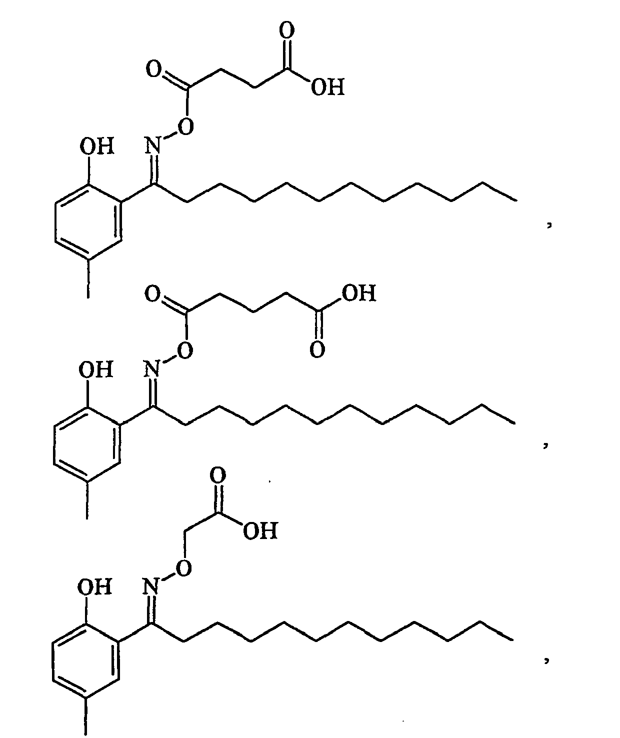

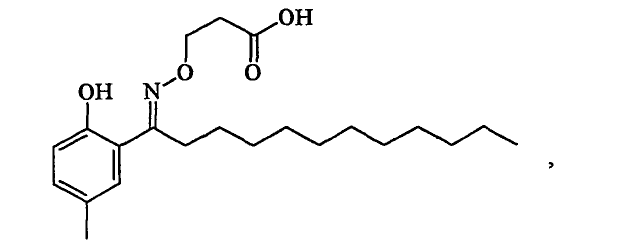

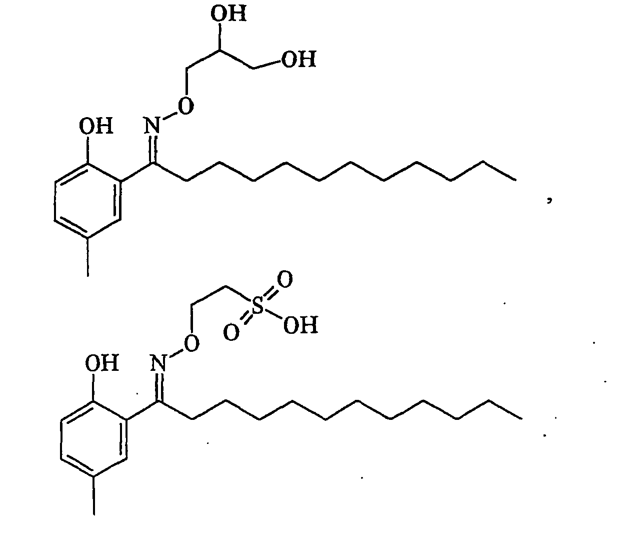

Besonders bevorzugt sind 2-Hydroxy-5-methyl-laurophenon-oxim sowie seine vorstehend genannten Oximderivate.

Die Komponente (a) liegt in der erfindungsgemäßen topischen Zusammensetzung in einer ausreichenden Menge vor, um für eine kosmetische oder dermatologische Anwendung geeignet zu sein. Üblicherweise ist die Komponente (a) in der erfindungsgemäßen topischen Zusammensetzung in einer Menge von 0,05 bis 5 Gew.%, vorzugsweise 0,02 bis 2 Gew.%, noch bevorzugter 0,05 bis 1,5 Gew.%, enthalten.

Die erfindungsgemäße topische Zusammensetzung enthält als Komponente (b) mindestens einen Emulgator, der wie vorstehend beschrieben aus speziellen Estern ausgewählt wird. Der Carbonsäurerest dieser Ester leitet sich von C₅₋₁₆-Säuren, vorzugsweise C₈₋₁₂-Säuren ab. Die Kohlenstoffkette des Carbonsäurerests kann gesättigt oder teilweise ungesättigt sein. Bevorzugte Beispiele des Carbonäurerests beinhalten Hexansäure, Caprylsäure, Caprinsäure, Laurinsäure, Myristinsäure, Palmitinsäure, Stearinsäure, Behensäure, Ölsäure, Linolsäure und Mischungen davon, z.B. Kokosfettsäure (deren Carbonsäurereste durch "Cocoyl" gekennzeichnet sind), die ein Gemisch aus den vorstehend genannten Fettsäuren darstellt.

Der Hydroxylrest des Esters kann sich von Monomeren, Dimeren oder Trimeren der Milchsäure oder eines ihrer Salze ableiten. Vorzugsweise wird ein Monomeres oder Dimeres der Milchsäure eingesetzt. Es ist weiterhin bevorzugt, daß die Milchsäure in Salzform, d.h. als Lactylat, verwendet wird. Besonders bevorzugt sind Alkalimetall- und Erdalkalimetallsalze, wobei insbesondere Natriumsalze hervorzuheben sind. Außerdem läßt sich der Hydroxylrest des Esters aus einem Polyglycerin aus 2 bis 10 Molekülen Glycerin ableiten. Dabei liegen pro mol Polyglycerin 1 bis 3 mol Carbonsäure vor. Besonders bevorzugt liegen pro mol Polyglycerin 2 bis 3 mol Carbonsäure vor.

Beispiele der Komponente (b), die in der erfindungsgemäßen topischen Zusammensetzung enthalten ist, beinhalten die Dispergierhilfsmittel, die in der DE-A-197 22 405 Spalte 2, Zeilen 38 bis 56 sowie in den Beispielen offenbart werden. Bevorzugt sind Polyglycerin-10-tricaprylat, Polyglycerin-10-trilaurat, Polyglycerin-2-oleat, Natriumlauryllactat, Natriumcocoyllactat, Caprin/Caprylsäuretriglycerid und Mischungen davon. Besonders bevorzugt sind Polyglycerin-2-oleat und Natriumcocoyllactylat.

Die Komponente (b) liegt in der erfindungsgemäßen topischen Zusammensetzung in einer ausreichenden Menge vor, um den Wirkstoff (Komponente (a)) in eine flüssig-kristalline (LC)-Phase zu integrieren und in diesem Zustand zu stabilisieren. Üblicherweise ist die Komponente (b) in der erfindungsgemäßen topischen Zusammensetzung in einer Menge von 0,5 bis 30 Gew.%, vorzugsweise 0,5 bis 20 Gew.%, noch bevorzugter 1 bis 10 Gew.%, enthalten.

Um die Stabilität der erfindungsgemäßen topischen Zusammensetzung in bezug auf eine verbesserte Löslichkeit und eine Vermeidung des Auskristallisierens des Wirkstoffes (Komponente (a)) weiter zu verbessern und gleichzeitig die Stabilität der erfindungsgemäßen topischen Zusammensetzung an sich zu gewährleisten, enthält die erfindungsgemäße topische Zusammensetzung vorzugsweise weiterhin als Komponente (c) mindestens einen Coemulgator, ausgewählt aus Glycerin- und Sorbitanesterderivaten sowie Cetearylalkohol und Esterderivaten davon und Mischungen dieser Verbindungen. Diese Coemulgatoren wirken sich nicht störend auf den Aufbau von flüssig-kristallinen Gelnetzwerken, die das Grundgerüst der erfindungsgemäßen topischen Zusammensetzung bilden sollten, aus. Die Glycerin-, Sorbitan- und Cetearylesterderivate leiten sich üblicherweise von Estern ab, deren Carbonsäurereste sich von C₅₋₁₆-Säuren herleiten, deren Kohlenstoffketten gesättigt oder teilweise ungesättigt sein können. Besonders bevorzugt sind davon Glycerinstearat, Sorbitanstearat, Sorbitanisostearat, Sorbitandiisostearat, Sorbitandioleat, Sorbitandistearat, Sorbitanlaurat, Sorbitanpalmitat, Sorbitansesquiisostearat, Sorbitansesquioleat, Sorbitantriisostearat, Sorbitantrioleat, Sorbitantristearat, Cetearyloctanoat, Ceterarylpalmitat, Cetearylisononanoat und Mischungen davon.

Die Komponente (c) liegt in der erfindungsgemäßen topischen Zusammensetzung in einer geeigneten Menge vor, um die topische Zusammensetzung weiter zu stabilisieren. Üblicherweise ist die Komponente (c) in einer Menge von 0,1 bis 40 Gew.%, vorzugsweise 0,5 bis 15 Gew.%, noch bevorzugter 1 bis 10 Gew.-%, in der erfindungsgemäßen topischen Zusammensetzung enthalten.

Zu einer weiteren Verbesserung der Löslichkeit des Wirkstoffes in der erfindungsgemäßen topischen Zusammensetzung ist vorzugsweise weiterhin als Komponente (d) mindestens ein lipophiles Lösungsmittel enthalten. Übliche lipophile Lösungsmittel, die für eine topische Formulierung geeignet sind, beinhalten Dimethicon, Cyclomethicon, Mineralöl, Isostearylisostearat, Octylpalmitat, Propylenglycol/Dicaprat/Dicaprylat, C₁₂₋₁₅-Alkylbenzoat, Octyldecanol, Etherderivate von Cetylalkohol, wie Ceteth-1, Ceteth-2, Ceteth-3, Ceteth-4, Ceteth-5, Ceteth-6 und Ceteth 10, Ethylbutylacetylaminopropionat, Ethanol, Isopropanol, Isopropylmyristat und Mischungen davon. Davon sind Ethylbutylacetylaminopropionat, Ethanol, Isopropanol, Isopropylmyristat und Mischungen davon besonders bevorzugt. Die Zugabe des lipophilen Lösungsmittels kann die Löslichkeit des Wirkstoffes erhöhen, so daß der Anteil an Wirkstoff (Komponente (a)) innerhalb der erfindungsgemäßen topischen Zusammensetzung erhöht werden kann. Bei Zusatz der Komponente (d) liegt die Wirkstoffmenge in der erfindungsgemäßen topischen Zusammensetzung vorzugsweise bei 0,01 bis 30 Gew.-%.

Die Komponente (d) liegt in der erfindungsgemäßen topischen Zusammensetzung üblicherweise in einer ausreichenden Menge vor, um die Löslichkeit des Wirkstoffes zu verbessern. Die Komponente (d) liegt vorzugsweise in einer Menge von 0,1 bis 20 Gew.%, noch bevorzugter 0,3 bis 17 Gew.%, vor.

Es ist weiterhin bevorzugt, daß in der erfindungsgemäßen topischen Zusammensetzung als Komponente (e) mindestens ein Hilfsstoff, ausgewählt aus Antioxidantien und UV-Filtern, enthalten ist.

Neben den bekannten Wirkungen der Antioxidantien und UV-Filter, wie einem Schutz vor Zellschädigung durch Radikale bzw. einem Schutz vor UV-Strahlung und deren schädlicher Wirkung, können die Antioxidantien und/oder UV-Filter den enthaltenen Wirkstoff (Komponente (a)) weiter stabilisieren. Dies wirkt sich z.B. in vorteilhafter Weise in einer Erhöhung der Lagerstabilität der erfindungsgemäßen topischen Zusammensetzung aus.

In den erfindungsgemäßen topischen Zusammensetzungen können die aus der Fachliteratur bekannten Antioxidantien enthalten sein, z.B. Flavonoide, Coumaranone, Aminosäuren (z.B. Glycin, Histidin, Tyrosin, Tryptophan) und deren Derivate, Imidazole, (z.B. Urocaninsäure) und deren Derivate, Peptide, wie D,L-Carnosin, D-Carnosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin), ferner (Metall-) Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat) sowie Koniferylbenzoat des Benzoeharzes, Rutinsäure und deren Derivate, α-Glycosylrutin, Ferulasäure, Furfurylidenglucitol, Carnosin, Butylhydroxyltoluol (BHT), Butylhydroxyanisol, Nordohydroguajaretsäure, Trihydroxybutyrophenon, Hamsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen topischen Zusammensetzungen geeignet. Bekannte und käufliche Mischungen sind beispielsweise Mischungen, enthaltend als aktive Inhaltsstoffe Lecithin, L-(+)-Ascorbylpalmitat und Zitronensäure (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

In einer bevorzugten Ausführungsform der Erfindung enthält die erfindungsgemäße topische Zusammensetzung als Antioxidans Butylhydroxytoluol.

In einer weiteren bevorzugten Ausführungsform enthält die erfindungsgemäße topische Zusammensetzung als Antioxidans eine oder mehrere Verbindungen, ausgewählt aus Flavonoiden und/oder Coumaranonen.

Als Flavanoide werden die Glycoside von Flavanonen, Flavonen, 3-Hydroxyflavonen (= Flavanolen), Auronen, Isoflavonen und Rotenoiden aufgefaßt (Römpp Chemie Lexikon, Band 9, 1993). Im Rahmen der vorliegenden Erfindung werden hierunter jedoch auch die Aglykone, d.h. die zuckerfreien Bestandteile, und die Derivate der Flavonoide und der Aglykone verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavanone sind durch folgende Grundstruktur gekennzeichnet:

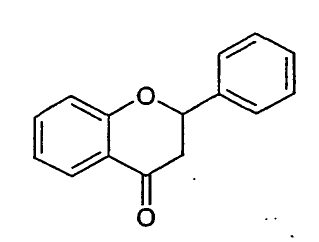

Die Flavone sind durch folgende Grundstruktur gekennzeichnet:

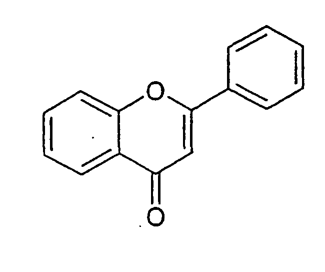

Die 3-Hydroxyflavone (Flavonole) sind durch folgende Grundstruktur gekennzeichnet:

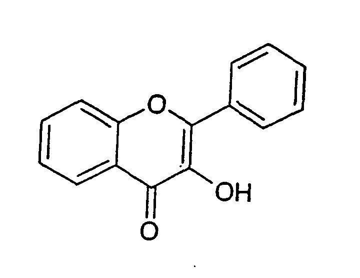

Die Isoflavone sind durch folgende Grundstruktur gekennzeichnet:

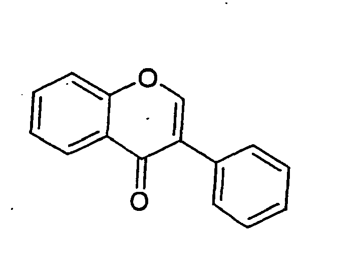

Die Aurone sind durch folgende Grundstruktur gekennzeichnet:

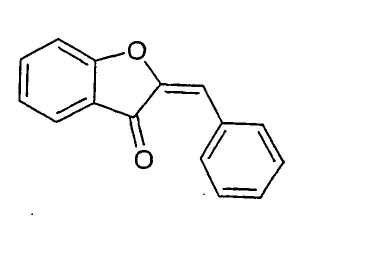

Die Coumaranone sind durch folgende Grundstruktur gekennzeichnet:

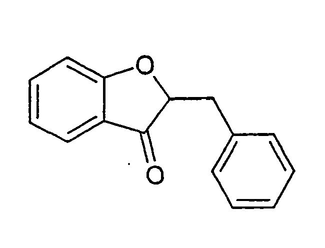

Vorzugsweise werden die Flavonoide und Coumaranone ausgewählt aus den Verbindungen der Formel (1): 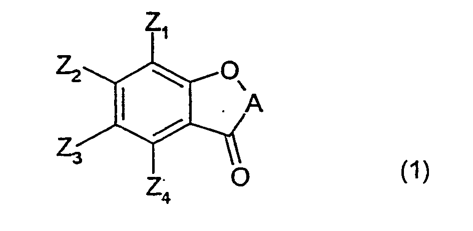 worin bedeuten:
- Z₁ bis Z₄: jeweils unabhängig voneinander H, OH, Alkoxy, Hydroxyalkoxy, Mono- oder Oligoglycosidreste, wobei die Alkoxy- und Hydroxyalkoxygruppen verzweigt und unverzweigt sein und 1 bis 18 C-Atome aufweisen können und wobei an die Hydroxygruppen der genannten Reste auch Sulfat oder Phosphat gebunden sein kann,
- A: ausgewählt wird aus der Gruppe, bestehend aus den Teilformen (1A), (1 B) und (1C) 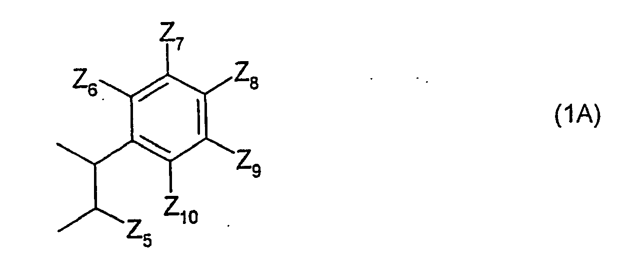 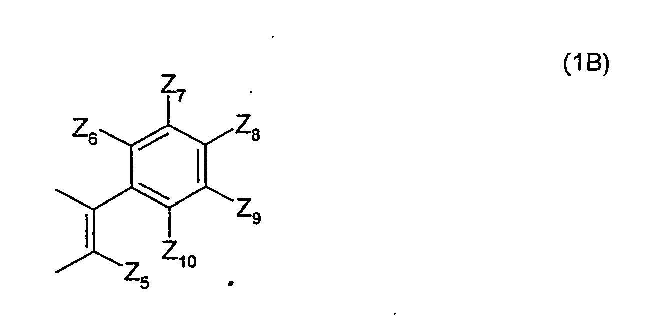 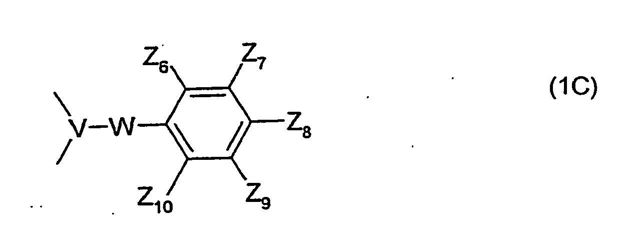
- Z₅: H, OH oder OR,
- R: einen Mono- oder Oligoglycosidrest,
- Z₆ bis: Z₁₀ die Bedeutung der Reste Z₁ bis Z₄ besitzen, und

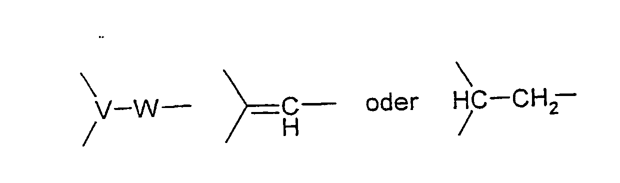

Die Alkoxygruppen sind vorzugsweise linear und besitzen 1 bis 12, vorzugsweise 1 bis 8 C-Atome. Diese Gruppen entsprechen somit der Formel -O-(CH₂)ₘ-H, wobei m 1,2,3,4,5,6,7 oder 8 und insbesondere 1 bis 5 bedeutet.

Die Hydroxyalkoxygruppen sind vorzugsweise linear und besitzen 2 bis 12, vorzugsweise 2 bis 8 C-Atome. Diese Gruppen entsprechen somit der Formel -O-(CH₂)ₙ-OH, wobei n 2,3,4,5,6,7 oder 8, insbesondere 2 bis 5 und besonders bevorzugt 2 bedeutet.

Die Mono- und Oligoglycosidreste sind vorzugsweise aus 1 bis 3 Glycosideinheiten aufgebaut. Vorzugsweise werden diese Einheiten ausgewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl, sind gegebenenfalls vorteilhaft zu verwenden. Es kann auch erfindungsgemäß vorteilhaft sein, Pentosylreste zu verwenden.

In einer bevorzugten Ausführungsform besitzen
- Z₁ und Z₃: die Bedeutung H,
- Z₂ und Z₄: eine andere Bedeutung als H, insbesondere bedeuten sie OH, Methoxy, Ethoxy oder 2-Hydroxyethoxy,
- Z₅: die Bedeutung H, OH oder einen Glycosidrest, der aus 1 bis 3, vorzugsweise aus 1 oder 2, Glycosideinheiten aufgebaut ist.
- Z₆, Z₉ und Z₁₀: die Bedeutung H, und
- Z₇ und Z₈: eine andere Bedeutung als H, insbesondere bedeuten sie OH, Methoxy, Ethoxy oder 2-Hydroxyethoxy.

In einer weiteren bevorzugten Ausführungsform, insbesondere, wenn die Wasserlöslichkeit der Flavonoide und Coumaranone gesteigert werden soll, ist an die Hydroxyguppen eine Sulfat- oder Phosphatgruppe gebunden. Geeignete Gegenionen sind beispielsweise die Ionen der Alkali- oder Erdalkalimetalle, wobei diese z.B. aus Natrium oder Kalium ausgewählt werden.

In einer weiteren bevorzugten Ausführungsform werden die Flavonoide ausgewählt aus folgenden Verbindungen: 4,6,3',4'-Tetrahydroxyauron, Quercetin, Rutin, Isoquercetin, Anthocyanidin (Cyanidin), Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfaten und Phosphaten.

Unter den Flavonoiden sind insbesondere Rutin und Troxerutin bevorzugt. Besonders bevorzugt ist Troxerutin.

Unter den Coumaranonen ist 4,6,3',4'-Tetrahydroxybenzylcoumaranon-3 bevorzugt.

Die Antioxidationsmittel werden in der Regel in einer Menge von 0,001 bis 5 Gew.%, vorzugsweise 0,5 bis 5 Gew.% in die erfindungsgemäßen topischen Zusammensetzungen eingearbeitet.

Als geeignete organische UV-Filter kommen alle dem Fachmann bekannten UVA- als auch UVB-Filter in Frage. Für beide UV-Bereiche gibt es viele aus der Fachliteratur bekannte und bewährte Substanzen, z.B.

Benzylidenkampferderivate, wie
- 3-(4'-Methylbenzyliden)-dl-kampfer (z.B. Eusolex® 6300),
- 3-Benzylidenkampfer (z.B. Mexoryl® SD),
- Polymere von N-{(2 und 4)-[(2-oxobom-3-yliden)methyl]benzyl}acrylamid (z.B Mexoryl® SW),
- N,N,N-Trimethyl-4-(2-oxoborn-3-ylidenmethyl)anilinium-methylsulfat (z.B. Mexoryl® SK) oder
- α-(2-Oxoborn-3-yliden)toluol-4-sulfonsäure (z.B. Mexoryl® SL),

Benzoyl- oder Dibenzoylmethane, wie
- 1-(4-tert-Butylphenyl)-3-(4-methoxyphenyl)propan-1,3-dion (z.B. Eusolex® 9020) oder
- 4-Isopropyldibenzoylmethan (z.B. Eusolex® 8020),

Benzophenone, wie
- 2-Hydroxy-4-methoxybenzophenon (z.B. Eusolex® 4360) oder
- 2-Hydroxy-4-methoxybenzophenon-5-sulfonsäure und ihr Natriumsalz (z.B. Uvinul® MS-40),

Methoxyzimtsäureester; wie
- p-Methoxyzimtsäure-2-ethylhexylester (z.B. Eusolex® 2292),
- p-Methoxyzimtsäureisopentylester, z.B. als Gemisch der Isomere (z.B. Neo Heliopan® E 1000),

Salicylatderivate, wie
- 2-Ethylhexylsalicylat (z.B. Eusolex® OS),
- 4-Isopropylbenzylsalicylat (z.B. Megasol® ) oder
- 3,3,5-Trimethylcyclohexylsalicylat (z.B. Eusolex® HMS),

4-Aminobenzoesäure und Derivate davon, wie
- 4-Aminobenzoesäure,
- 4-(Dimethylamino)benzoesäure-2-ethylhexylester (z.B. Eusolex® 6007),
- ethoxylierte 4-Aminobenzoesäureethylester (z.B. Uvinul® P25),
und weitere Substanzen, wie
- 2-Cyano-3,3-diphenylacrylsäure-2-ethylhexylester (z.B. Eusolex® OCR),
- 2-Phenylbenzimidazol-5-sulfonsäure sowie ihre Kalium-, Natrium- und Triethanolaminsalze (z.B. Eusolex® 232),
- 3,3'-(1,4-Phenylendimethylen)-bis-(7,7-dimethyl-2-oxobicyclo[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150).

Diese organischen UV-Filter werden in der Regel in einer Menge von 0,5 bis 10 Gew.%, vorzugsweise 1 bis 8 Gew.%, in die erfindungsgemäßen topischen Zusammensetzungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole® ),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω[trimethylsilyl)oxy]poly[oxy(dimethyl] [und ca. 6% methyl[2-[p-[2,2-bis(ethoxycarbonyl]vinyl]phenoxy]-1-methylenethyl] und ca. 1,5% methyl[3-[p-[2,2-bis(ethoxycarbonyl)vinyl)phenoxy)-propenyl) und 0,1 bis 0,4% (methylhydrogen]silylen]] (n≈60) (z.B. Parsol® SLX,
- 2,2'-Methylen-bis-(6-(2H-benzotriazol-2-yl)-4-(1,1,33-tetramethylbutyl)phenol (z.B. Tinosorb® M),
- 2,2'-(1,4-Phenylen)bis-1 H-benzimidazol-4,6-disulfonsäure, Mononatriumsalz,
- 2,2'-(1,4-Phenylen)bis-1 H-benzimidazol-5-sulfonsäure, Mononatriumsalz,
- 2,2'-(1,4-Phenylen)bis-1 H-benzimidazol-4,6-disulfonsäure, Monokaliumsalz,
- 2,4-bis-{[4-(2-Ethyl-hexyloxy)-2-hydroxyl]-phenyl}-6-(4-methoxyphenyl)-1,3,5-triazin (z.B. Tinosorb® S).

Diese organischen Filter werden in der Regel in einer Menge von 0,5 bis 20 Gew.%, vorzugsweise 1 bis 15 Gew.%, in die erfindungsgemäßen topischen Zusammensetzungen eingearbeitet.

Als anorganische UV-Filter sind solche aus der Gruppe der Titandioxide, z.B. gecoatetes Titandioxid (z.B. Eusolex® T-2000 oder Eusolex® T-Aqua), Zinkoxide (z.B. Sachtotec® ), Eisenoxide oder auch Ceroxide denkbar. Diese anorganischen UV-Filter werden in der Regel in einer Menge von 0,5 bis 20 Gew.%, vorzugsweise 2 bis 10 Gew.%, in die erfindungsgemäßen topischen Zusammensetzungen eingearbeitet.

Bevorzugte UV-Filter sind Zinkoxid, Titandioxid, 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze.

Besonders bevorzugte UV-Filter sind Zinkoxid und Titandioxid.

Unter den Titandioxid enthaltenden erfindungsgemäßen topischen Zusammensetzungen sind diejenigen bevorzugt, die neben Titandioxid zusätzlich einen oder mehrere weitere UV-Filter, ausgewählt aus 3-(4'-Methylbenzyliden)-dl-kampfer, 1-(4-tert-Butylphenyl)-3-(4-methoxyphenyl)propan-1,3-dion, 4-lsopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze, enthalten.

Unter diesen Zusammensetzungen sind diejenigen insbesondere bevorzugt, die neben Titandioxid zusätzlich die UV-Filter 2-Hydroxy-4-methoxybenzophenon und/oder Methoxyzimtsäureoctylester enthalten.

Zur Verbesserung des Hautschutzes und einer Immunsuppression der Haut ist die Kombination von Aryloximen mit Ectoin und Ectoinderivaten besonders wirksam.

Je nach Anwendung kann die erfindungsgemäße topische Zusammensetzung gegebenenfalls weitere Hilfs- und/oder Trägerstoffe, wie Trägermittel, Konservierungsstoffe, Stabilisatoren, Lösungsmittel, Vitamine, Färbemittel, Geruchsverbesserer, Filmbildner, Verdickungsmittel und Feuchthaltemittel, enthalten.

Als Anwendungsform der erfindungsgemäßen topischen Zusammensetzung seien z.B. genannt: Lösungen, Suspensionen, Emulsionen, Pasten, Salben, Gele, Cremes, Lotionen, tensidhaltige Reinigungspräparate und Öle.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silikone, Bentonite, Kieselsäure, Talkum und Zinkoxid, Xanthangummi, Glycerin, Carboxypolymethylen oder Gemische dieser Stoffe.

Lösungen und Emulsionen können die üblichen Trägerstoffe, wie Lösungsmittel, Lösungsvermittler und Emulgatoren, z.B. Wasser, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Polyethylenglykole, Xanthangummi, Glycerin, Carboxypolymethylen oder Gemische dieser Stoffe, enthalten.

Suspensionen können die üblichen Trägerstoffe, wie flüssige Verdünnungsmittel, z.B. Wasser oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth, Xanthangummi, Glycerin, Carboxypolymethylen oder Gemische dieser Stoffe, enthalten.

Tensidhaltige Reinigungsprodukte können die üblichen Trägerstoffe, wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarcosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe, enthalten.

Gesichts-und Körperöle können die üblichen Trägerstoffe, wie synthetische Öle, z.B. Fettsäureester, Fettalkohole, Silikonöle, natürliche Öle, wie Pflanzenöle und ölige Pflanzenauszüge, Paraffinöle, Lanolinöle oder Gemische dieser Stoffe, enthalten.

Die erfindungsgemäße topische Zusammensetzung wird nach dem vorstehend beschriebenen Verfahren hergestellt. Verschiedene Verfahrenstechniken können angewendet werden, um die Bestandteile der erfindungsgemäßen topischen Zusammensetzung möglichst innig zu vermischen. Je nach der erforderlichen Mischintensität werden ein oder mehrere Verfahren der Stoffvereinigung eingesetzt, die nacheinander oder parallel ablaufen können.

In der chemischen Verfahrenstechnik sind unter dem Begriff "Mischen" Grundoperationen zu verstehen, die der weitestgehenden Homogenisierung von Stoffen dienen. Es sollen Stoffströme so vereinigt werden, daß in Teilvolumina der entstehenden Mischung eine möglichst gleichmäßige Zusammensetzung der einzelnen Komponenten gegeben ist.

Eine Spezialform des Mischens stellt das Homogenisieren dar. Hierunter ist ein Vermischen von an sich nicht miteinander mischbaren Phasen zu verstehen. Unter Homogenisieren versteht man demnach ein Verändern des Verteilungszustandes und der Teilchengröße der inneren Phase von Emulsionen und Suspensionen, so daß mikroskopisch betrachtet ein homogenes System entsteht und sich die verteilte Phase ohne Einwirkung äußerer Kräfte nicht absetzt oder aufrahmt.

Unter Dispergieren ist ein Vermischen eines aus zwei oder mehreren Phasen bestehenden Stoffsystems zu verstehen, bei dem ein Stoff (disperse Phase) in einem anderen (Dispersionsmittel) in feinster Form verteilt (dispergiert) wird. Sowohl die Teilchen der dispersen Phase als auch das Dispersionsmittel können fest, flüssig oder gasförmig sein. Beispiele für Dispersionen sind Aerosole, Emulsionen, Suspensionen und Kolloide.

Eine andere in der Kosmetikherstellung übliche Art des Vermischens besteht im Emulgieren. Darunter ist ein Vermischen von zwei nicht oder nur wenig ineinander löslichen Flüssigkeiten zu verstehen, von denen die eine in der anderen fein verteilt wird. Die äußere Phase bezeichnet man als kontinuierliche Phase bzw. als Dispersionsmittel, die darin verteilte Flüssigkeit als die innere, diskontinuierliche oder disperse Phase. Kosmetische Emulsionen bestehen meistens aus einer wäßrigen polaren Phase und einer unpolaren Ölphase.

Unter Suspendieren wiederum ist das Verteilen sehr kleiner jedoch nicht molekularer Teilchen eines festen Stoffes oder einer Flüssigkeit zu verstehen. Suspensionen sind wie Emulsionen meist optisch trüb und neigen dazu, sich unter Einfluß der Schwerkraft abzusetzen.

Die vorstehend genannten Mischverfahren sind für die Herstellung der erfindungsgemäßen topischen Zusammensetzung geeignet. Besonders bevorzugt wird die erfindungsgemäße topische Zusammensetzung durch Homogenisieren, Dispergieren bzw. Emulgieren hergestellt.

Die Komponenten (a) und (b) werden vor dem Einarbeiten in einen Träger gemischt, um eine Phase A herzustellen. Dies dient dazu, die Löslichkeit und Stabilität der Komponente (a) in der erfindungsgemäßen topischen Zusammensetzung zu gewährleisten. Das Mischen wird vorzugsweise unter Rühren und bei einer erhöhten Temperatur durchgeführt. Die Temperatur während des Mischvorgangs beträgt vorzugsweise 60 bis 100°C, noch bevorzugter 70 bis 90°C.

Nach der Herstellung der Phase A wird diese in eine Phase B eingearbeitet. Die Art der Phase B, und insbesondere die Art des darin enthaltenen Trägers, sind abhängig von der Anwendungsform der erfindungsgemäßen topischen Zusammensetzung, die vorstehend aufgelistet werden. Folglich ist die Phase B vorzugsweise eine wäßrige Phase oder eine Ölphase.

Der Einarbeitungsschritt von Phase A in Phase B erfolgt vorzugsweise nach einem Hot/Hot-Prozeß, d.h. beide Phasen werden getrennt voneinander vor dem Einarbeitungsschritt erwärmt. Die Temperatur beträgt vorzugsweise 60 bis 100°C, noch bevorzugter 70 bis 90°C. Jedoch kann in einigen Fällen die Temperatur einer der Phasen, vorzugsweise der Phase B, 20 bis 30°C betragen. Dieses Verfahren wird als Hot/Cold-Prozeß bezeichnet. Es ist weiterhin möglich, daß beide Phasen mit einer Temperatur von 20 bis 30°C vorgelegt werden (Cold/Cold-Prozeß).

Die weiteren Komponenten, insbesondere Komponente (c) bis (d), die wahlweise in der erfindungsgemäßen topischen Zusammensetzung enthalten sind, können in Phase A und/oder Phase B vorliegen. Es ist ebenfalls möglich, daß diese Komponenten nach der Einarbeitung der Phase A in Phase B zugefügt werden.

Die erhaltene erfindungsgemäße topische Zusammensetzung eignet sich zur Prophylaxe, Pflege und/oder Behandlung von Hauterkrankungen und/oder Entzündungsreaktionen der Haut. Insbesondere seien die folgenden Hauterkrankungen und Entzündungsreaktionen der Haut genannt:
- irritative Dermatitis,
- toxische Dermatitis,
- allergische Erkrankungen der Haut bzw. der Hautadnexen,
- entzündliche Erkrankungen der Haut bzw. der Hautadnexen,
- UV-Dermatitis und
- unterschiedliche Ekzemformen.

Die Erfindung wird anhand der folgenden Beispiele näher erläutert.

### Beispiel 1

### Beurteilung der Löslichkeiten des binären Phasenverhaltens

2-Hydroxy-5-methyl-laurophenon-oxim wird durch Rühren bei ca. 90°C in einer Menge von 10 Gew.% jeweils in einen der folgenden Emulgatoren eingearbeitet:
(a) Polyglycerin-10-tricaprylat
(b) Polyglycerin-10-trilaurat
(c) Polyglyerin-2-oleat
(d) Natriumlauryllactylat
(e) Natriumcocoyllactylat.

Die erhaltenen Vormischungen wurden in einer Menge von 10 Gew.% in demineralisiertem Wasser als Träger eingearbeitet. Das Einarbeiten erfolgt unter Rühren bei 90°C.

Nach Abkühlen auf Raumtemperatur wurden diese Mischungen kontaktmikroskopisch untersucht. Dabei konnte herausgefunden werden, daß flüssig-kristalline Myelinstrukturen bei etwa 25°C gebildet wurden. Es konnte somit eine erfolgreiche liposomale Verkapselung des Wirkstoffes erreicht werden.

### Beispiel 2

### Beurteilung der Kompatibilität mit lipophilen Coemulqatoren

Die vorstehend genannten Vormischungen von Komponente (a) mit (b) wurden mit Coemulgatoren kombiniert, welche in Wasser flüssig-kristalline Gelnetzwerke aufbauen. Als lipophile Coemulgatoren wurden Glycerylstearat, Cetearylalkohol und Sorbitanstearat untersucht. Dabei wurden die folgenden Zusammensetzungen hergestellt:

Mischemulgatoren auf Basis von Polyglycerin-2-oleat:

| Rezeptur Nr. | 2-1 (Gew.%) | 2-2 (Gew.%) | 2-3 (Gew.%) |
|---|---|---|---|
| Phase A | | | |
| 2-Hydroxy-5-methyl-laurophenon-oxim | 2,25 | 2,25 | 2,25 |
| Rylo PG 29 (Polyglycerin-2-oleat) | 27,75 | 27,75 | 27,75 |
| Tegin M (Glycerylstearat) | 70,00 | 0,00 | 0,00 |
| Lanette O (Cetearylalkohol) | 0,00 | 70,00 | 0,00 |
| Span 60 (Sorbitanstearat) | 0,00 | 0,00 | 70,00 |
| Summe | 100,00 | 100,00 | 100,00 |

Mischemulgatoren auf Basis von Natriumcocoyllactylat:

| Rezeptur Nr. | 2-4 (Gew.%) | 2-5 (Gew.%) | 2-6 (Gew.%) |
|---|---|---|---|
| Phase A | | | |
| 2-Hydroxy-5-methyl-laurophenon-oxim | 2,25 | 2,25 | 2,25 |
| Pationic SCL (Natriumcocoyllactylat) | 27,75 | 27,75 | 27,75 |
| Tegin M (Glycerylstearat) | 70,00 | 0,00 | 0,00 |
| Lanette O (Cetearylalkohol) | 0,00 | 70,00 | 0,00 |
| Span 60 (Sorbitanstearat) | 0,00 | 0,00 | 70,00 |
| Summe | 100,00 | 100,00 | 100,00 |

Die Bestandteile der Phase A wurden miteinander vermischt, indem die Bestandteile unter Rühren auf 90°C erwärmt wurden und danach auf 25°C abgekühlt wurden. Die erhaltenen Gemische wurden zur Bildung flüssig-kristalliner Gelnetzwerke jeweils 10%ig in demineralisiertes Wasser (Phase B) eingearbeitet. Dabei wurde Phase A unter Rühren auf 90°C erwärmt. Gleichzeitig wurde Phase B auf 80°C erwärmt. Phase A wurde zu Phase B gegeben und das erhaltene Gemisch eine Minute lang homogenisiert. Die erhaltene Zusammensetzungen wiesen gegenüber herkömmlichen Zusammensetzungen mit 2-Hydroxy-5-methyl-laurophenon-oxim eine verbesserte Lagerstabilität auf.

### Beispiel 3

### Topische Zusammensetzung als O/W-Emulsion

Die im folgenden beschriebenen Phasen A und B wurden auf die gleiche Weise, wie in Beispielen 1 und 2 beschrieben, miteinander verbunden, um eine topische Zusammensetzung herzustellen.

Die folgenden Zusammensetzungen wurden hergestellt:

**Tabelle 1**

| Handelsname | Bezeichnung | 3-1 (Gew.%) | 3-2 (Gew.%) |
|---|---|---|---|
| Phase A | | | |
| Rylo PG-29 | Polyglycerin-2-oleat | 2,00 | 1,00 |
| Tegin M | Glycerinstearat | 1,00 | 1,00 |
| Span 60 | Sorbitanstearat | 3,00 | 3,00 |
| 2-Hydroxy-5-methyl-laurophenon-oxim | | 0,50 | 0,50 |
| Miglyol 812 | Caprin/Caprylsäuretriglycerid | 16,00 | 17,00 |

| Phase B | | | |
|---|---|---|---|
| Glycerin | | 6,00 | 6,00 |
| Keltrol | Xanthangummi | 0,30 | 0,30 |
| Demin. Wasser | | 71,20 | 71,20 |
| Summe | | 100,00 | 100,00 |

**Tabelle 2**

| Handelsname | Bezeichnung | 3-3 (Gew.%) |
|---|---|---|
| Phase A | | |
| Pationic SCL | Na-Cocoyllactylat | |
| Tegin M | Glycerinstearat | 1,00 |
| Span 60 | Sorbitanstearat | 3,00 |
| 2-Hydroxy-5-methyl-laurophenon-oxim | | 0,50 |
| Miglyol812 | Caprin/Caprylsäure-triglycerid | 16,00 |

| Phase B | | |
|---|---|---|
| Glycerin | | 6,00 |
| Keltrol | Xanthangummi | 0,30 |
| Demin. Wasser | | 71,20 |
| Summe | | 100,00 |

**Tabelle 3**

| Handelsname | Bezeichnung | 3-4 (Gew.%) | 3-5 (Gew.%) | 3-6 (Gew.%) | 3-7 (Gew.%) |
|---|---|---|---|---|---|
| Phase A* | | | | | |
| Biobase EP | Glycerinstearat, Cetearylalkohol, Na-Stearoyl-lactylat, Lecithin | 4,50 | 4,50 | 4,50 | 4,50 |
| Miglyol 812 | Caprin/Capryl-Säuretriglycerid | 15,25 | 15,00 | 14,75 | 14,50 |
| 2-Hydroxy-5-methyl-laurophenon-oxim | | 0,25 | 0,50 | 0,75 | 1,00 |

| Phase B | | | | | |
|---|---|---|---|---|---|
| ETD 2050 | Carbomer | 0,30 | 0,30 | 0,30 | 0,30 |
| Demin. Wasser | | 79,70 | 79,70 | 79,70 | 79,70 |
| Summe | | 100,00 | 100,00 | 100,00 | 100,00 |

| | | | | | |
|---|---|---|---|---|---|
| * Phasen A und B wurden jeweils auf 70°C erwärmt und bei dieser Temperatur miteinander vermischt. | | | | | |

Die erhaltenen Emulsionen zeigten über einen Zeitraum von vier Wochen keinerlei Kristallisation des Wirkstoffes bei Raumtemperatur, 40°C und 5 Tau/Gefrierzyklen zwischen -18°C und -40°C.

### Beispiel 4

### Topische Zusammensetzung als W/O-Emulsion

| | Rohstoff | INCI-Name | 4-1 Gew.% | 4-2 Gew.% | 4-3 Gew.% |
|---|---|---|---|---|---|
| | | | | | |
| A | Isolan PDI | Diisostearoyl Polyglyceryl-3-Diisostearat | 3,00 | 3,00 | 3,00 |
| | Paraffinöl, fl. | Mineral Oil | 17,00 | 17,00 | 17,00 |
| | Isopropylmyristat | Isopropyl Myristate | 5,00 | 5,00 | 5,00 |
| | Bienenwachs | Beeswax | 0,20 | 0,20 | 0,20 |
| | Cutina HR | Hydrogenated Castor Oil | 0,30 | 0,30 | 0,30 |
| | | | | | |
| B | Demin. Wasser | Aqua | ad100 | ad100 | ad100 |
| | Glycerin (87%) | Glycerin | 4,00 | 4,00 | 4,00 |
| | Magnesiumsulfat | Magnesium Sulfate | 1,00 | 1,00 | 1,00 |
| | Germaben II-E | Propylene Glycol, Diazolidinyl Urea, Methylparaben, Propylparaben Urea | 10,00 | 10,00 | 10,00 |
| | | | | | |
| C | 2-Hydroxy-5-methyl- laurophenon-oxim | 2-Hydroxy-5-methyl- laurophenon-oxim | 1,00 | 2,00 | 4,00 |

Die Phasen A und B wurden auf 75°C erwärmt. B wurde unter Rühren zu A gegeben. Anschließend wurde das Gemisch bei 9000upm 2 Min. mit dem Turrax homogenisiert. Das erhaltene Gemisch wurde auf 30 bis 35°C abgekühlt, und C wurde eingerührt.

### Beispiel 5

### Topische Zusammensetzung als W/O-Emulsion

| | Rohstoff | INCI-Name | 5-1 Gew.% | 5-2 Gew.% | 5-3 Gew.% |
|---|---|---|---|---|---|
| | | | | | |
| A | Arlacel1689 | | 6,00 | 6,00 | 6,00 |
| | Paraffinöl, fl. | Mineral Oil | 10,00 | 10,00 | 10,00 |
| | Miglyol 812 | Caprylic/Capric Triglyceride | 5,00 | 5,00 | 5,00 |
| | | | | | |
| B | Demin. Wasser | Aqua | ad100 | ad100 | Ad100 |
| | Glycerin (87%) | Glycerin | 4,00 | 4,00 | 4,00 |
| | Magnesiumsulfat | Magnesium Sulfate | 0,50 | 0,50 | 0,50 |
| | Germaben II-E | Propylene Glycol, Diazolidinyl Urea, Methylparaben, Propylparaben | 0,5 | 0,5 | 0,5 |
| | Harnstoff | Urea | 10,00 | 10,00 | 10,00 |
| | | | | | |
| C | 2-Hydroxy-5-methyl-laurophenon-oxim | 2-Hydroxy-5-methyl-laurophenon-oxim | 1,00 | 2,00 | 4,00 |

Die Phasen A und B wurden auf 75°C erwärmt. B wurde unter Rühren zu A gegeben. Anschließend wurde das Gemisch bei 9000upm 2 Min. mit dem Turrax homogenisiert. Das erhaltene Gemisch wurde auf 30 bis 35°C abgekühlt, und C wurde eingerührt.

### Beispiel 6

### Topische Zusammensetzung als W/O-Emulsion

| | Rohstoff | INCI-Name | 6-1 Gew.% | 6-2 Gew.% | 6-3 Gew.% |
|---|---|---|---|---|---|
| | | | | | |
| A | Paraffin, dickflüssig | Mineral Oil | 36,9 | 36,9 | 36,9 |
| | Migylol 812 | Caprylic/Capric Triglyceride | 10 | 10 | 10 |
| | Wachs, gebleicht (1.11544) | Cera Alba | 4 | 4 | 4 |
| | Cutina CP | Cetyl Palmitate | 3 | 3 | 3 |
| | | | | | |
| B | Demin. Wasser | Aqua | ad100 | ad100 | ad100 |
| | Propylenglycol (1.07478) | Propylene Glycol | 10 | 10 | 10 |
| | Germaben II-E | Propylene Glycol, Diazolidinyl Urea, Methylparaben, Propylparaben | 0,5 | 0,5 | 0,5 |
| | Harnstoff (108486) | Urea | 10 | 10 | 10 |
| | | | | | |
| C | 2-Hydroxy-5-methyl-laurophenon-oxim | 2-Hydroxy-5-methyl-laurophenon-oxim | 1 | 2 | 4 |

Die Fettphase (A) und die Wasserphase (B) wurden im Wasserdampfbad getrennt und auf 75°C erwärmt. Die Phase B wurde unter Rühren langsam zu A gegeben (Propellerrührer, 500 upm, Dauer der Zugabe ca. 1 Min.). Anschließend wurde die Emulsion homogenisiert (Ultra Turax, 9000 upm, 2 Min.). Das erhaltene Gemisch wurde unter Rühren auf ca. 35°C abgekühlt, und Phase C wurde zugegeben. Dabei wurde die Rührgeschwindigkeit so angepaßt, daß die Emulsion ständig in homogener Bewegung war und keine Luft eingerührt wurde, z.B. mit einem Propellerrührer, 500 upm.

### Beispiel 7

### Prüfung der Beeinflussung UV-Licht induzierter Erytheme durch topische Vor- und Nachbehandlung mit einer wirkstoffhaltigen Formulierung im Vergleich zum Placebo

Zielgrößen der vorliegenden Prüfung sind die visuelle Beurteilung zur Bestimmung der Beeinflussung UV-Licht induzierter Erytheme verschiedener Ausprägung durch eine topische wirkstoffhaltige Formulierung im Vergleich zum Placebo, deren Anwendung sowohl vor als auch nach der Erytheminduktion erfolgte.

Die Prüfung wurde als Doppelblindprüfung durchgeführt. An der Prüfung nahmen dreizehn Probanden teil, die alle die Prüfung korrekt und vollständig absolvierten. Pro Proband wurden vier Prüfareale je 37 cm² auf der unteren Rückenhälfte definiert, wobei zwei Prüfareale vor der Erytheminduktion mit Prüfpräparaten in Quantitäten von je 2,0 mg/cm² vorbehandelt wurden. Auf zwei weiteren Prüfarealen erfolgte die Behandlung mit den Prüfpräparaten in der gleichen Menge erst nach der Erytheminduktion.

Die Prüfareale jedes Probanden wurden daher wie folgt zusammengefasst:
Prüfareal 1: Vorbehandlung UV-Exposition, Prüfpräparat Placebo
Prüfareal 2: Vorbehandlung UV-Exposition, Prüfpräparat Wirkstoff
Prüfareal 3: Nachbehandlung UV-Exposition, Prüfpräparat Placebo
Prüfareal 4: Nachbehandlung UV-Exposition, Prüfpräparat Wirkstoff.

Die Prüfpräparate besaßen die folgenden Inhaltsstoffe und wurden als O/W-Emulsion formuliert.

Inhaltsstoffe/Formulierung:

| Rohstoff | Charge | INCI-Name | Gew.% | |
|---|---|---|---|---|
| | | | Prüfpräparat Wirkstoff | Prüfpräparat Placebo |
| Biobase EP | 393/97 | Glycerin Sterarate, Cetearyl Alcohol, Sodium Stearoyl Lactylate, Lecithin | 4,50 | 4,50 |
| Miglyol 812 | K26777507 | Caprylic/CapricTriglyceride | 15,00 | 16,00 |
| 2-Hydroxy-5- methyllauro-phenonoxim | 99/FA/024 | (2-Hydroxy-5-methyl-laurophenonoxime) | 1,00 | - |
| ETD 2050 | X418003 | Carbomer | 0,30 | 0,30 |
| Demin. Wasser | | Aqua | ad 100 | ad 100 |
| Glycerin (87%ig) | K26164291907 | Glycerin | 3,00 | 3,00 |
| Germaben II-E | GBTE-204 | Propylene Glycol (and) Diazolidinyl Urea | 0,50 | 0,50 |
| NaOH (10%ig) | 70164760 | Sodium Hydroxide | 0,60 | 0,50 |

Die Prüfareale 1 und 2 wurden insgesamt fünfmal vor der Erytheminduktion mit dem jeweiligen Prüfpräparat behandelt. Diese Vorbehandlung erfolgte am 1. und 2. Tag vor der Erytheminduktion zweimal täglich sowie 1 Stunde vor Beginn der Erytheminduktion.

Die Nachbehandlung der entsprechenden Prüfareale 3 und 4 erfolgte insgesamt viermal und begann unmittelbar nach der UV-Exposition. Sie wurde am Tag der Erytheminduktion einmal und am darauffolgenden Tag zweimal wiederholt.

Zur Induktion der UV-Erytheme wurden die Prüfareale mittels eines Sonnensimulators (SOL 500, Dr. Hönle) mit folgenden Dosen bestrahlt: 0; 0,5; 1,0; 1,25; 1,5 und 1,75 MED (minimale erythemale Dosis). Die Bestimmung der MED jedes Probanden erfolgte zwei Tage vor der UV-Exposition der Prüfareale auf einem separaten Areal auf dem Rücken. Demzufolge entsprachen die Bestrahlungszeiten der ermittelten individuellen MED, so dass eine Bestrahlung mit den vorstehend genannten Dosen gewährleistet wurde. 24 Stunden nach der Erytheminduktion auf den einzelnen Prüfarealen der dreizehn Probanden erfolgte die visuelle Beurteilung der Erytheme entsprechend einer Bewertungsskala:

| **sichtbare Hautveränderung** | **Bewertung** |
|---|---|
| kein Eindruck eines Erythems | 0 |
| zweifelhaftes Erythem | 0,5 |
| schwaches Erythem | 1/1,5 |
| mäßiges Erythem | 2/2,5 |
| starkes Erythem | 3/3,5 |
| starkes Erythem mit Ödembildung | 4 |

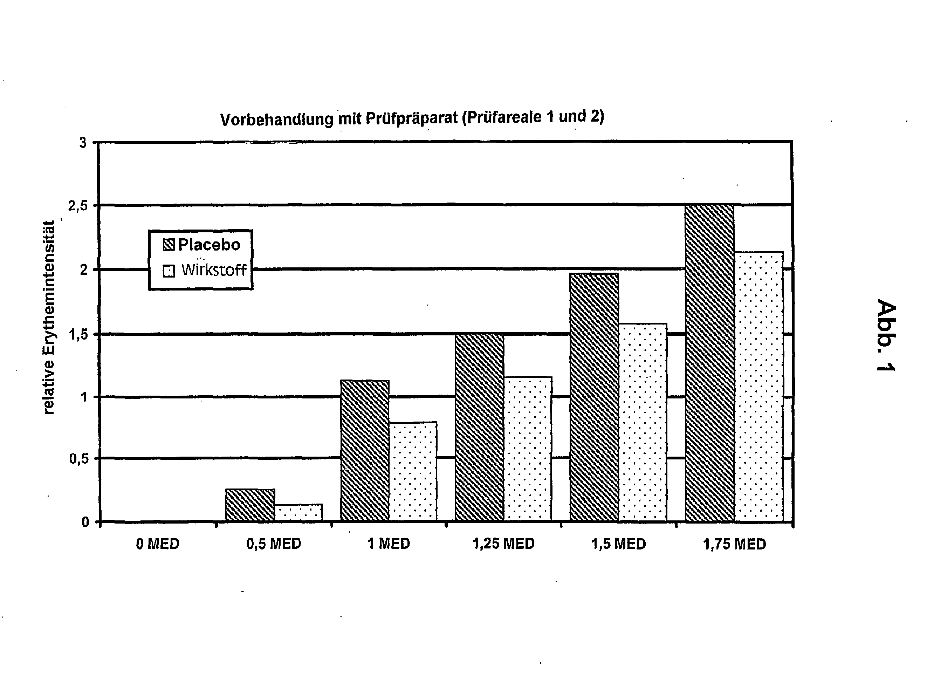

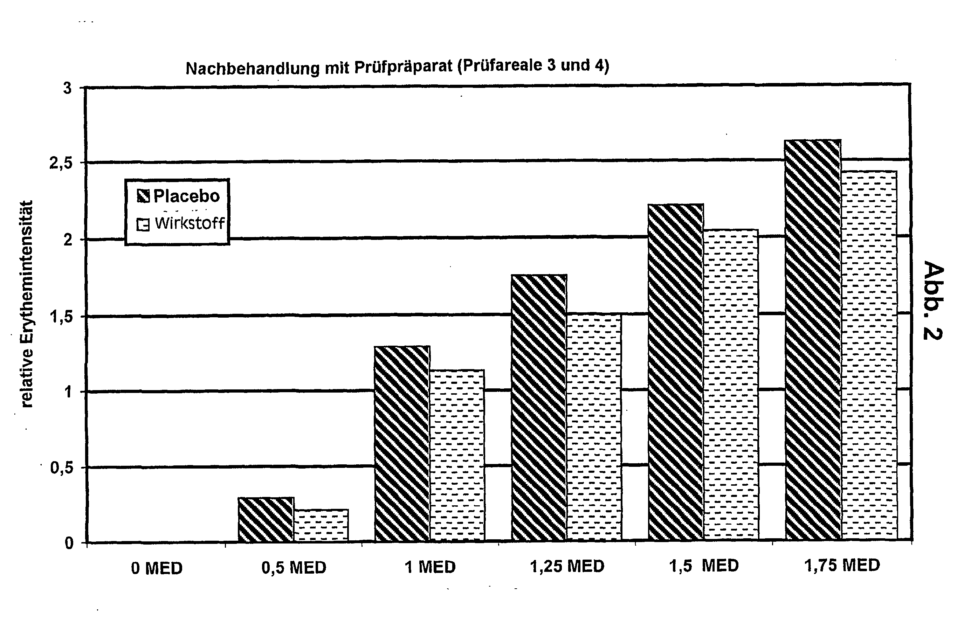

Aus den erhaltenen Bewertungen der Probanden wurden Mittelwerte gebildet. Diese Mittelwerte der visuellen Beurteilung der Erytheme werden in den Abbildungen 1 und 2 graphisch gezeigt und werden in der folgenden Tabelle zusammengefasst.

| **MED** | **Prüfareal 1** | **Prüfareal 2** | **Prüfareal 3** | **Prüfareal 4** |
|---|---|---|---|---|
| 0 | 0 | 0 | 0 | 0 |
| 0,5 | 0,25 | 0,13 | 0,29 | 0,21 |
| 1,0 | 1,13 | 0,79 | 1,29 | 1,13 |
| 1,25 | 1,5 | 1,16 | 1,75 | 1,5 |
| 1,5 | 1,96 | 1,58 | 2,21 | 2,04 |
| 1,75 | 2,5 | 2,13 | 2,63 | 2,42 |

Unter Beachtung der visuellen Begutachtung ergaben sich statistisch signifikante Unterschiede zwischen der Wirkstoff- und der Placebobehandlung bei der Beurteilung der UV-Licht induzierten Erytheme, wobei die Wirkstoffbehandlung den Grad der Erytheme sowohl bei der Vor- als auch bei der Nachbehandlung reduzierte.

Die verzeichneten Unterschiede betreffen einen Unterschied von 0,5 Bewertungspunkten der visuellen Bewertungsskala.

Auch durchgeführte Mexameter-Messungen ergaben eine signifikant positive Wirkung des Wirkstoffes sowohl bei der Vor- als auch bei der Nachbehandlung UV-Licht induzierter Erytheme (nicht gezeigt).

## Patentansprüche

1. Topische Zusammensetzung, enthaltend
(a) mindestens ein Aryloxim der Formel (I) und
(b) mindestens einen Emulgator, 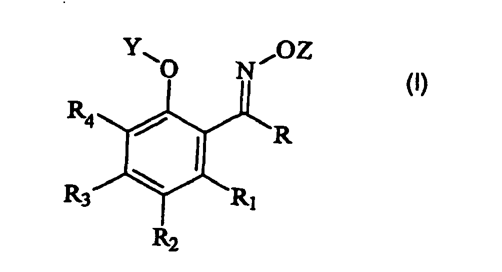 worin bedeuten:
Y, Z unabhängig voneinander H, C₁₋₁₈-Alkyl, C₂₋₁₈-Alkenyl, C₂₋₁₈₋Carboxyalkyl, C₃₋₁₈-Carboxyalkenyl oder C₂₋₁₈-Alkanoyl;
R C₁₋₁₈-Alkyl, C₂₋₁₈-Alkenyl, C₃₋₈-Cycloalkyl, Aryl, Aralkyl, Heteroaryl, Heteroaralkyl oder kondensierte Systeme;
R₁, R₂, R₃, R₄ unabhängig voneinander H, C₁₋₁₂-Alkyl, C₂₋₁₂-Alkenyl, C₁₋₁₂-Alkoxy, C₃₋₈-Cycloalkoxy, Aryl, Aryloxy, Aralkyl, Heteroaryl, Heteroaralkyl, Carboxy, Hydroxy, Chlor, Dialkylamin oder Sulfonyl,
**dadurch gekennzeichnet, daß** die Komponente (b) ausgewählt wird aus der Gruppe, bestehend aus mindestens einem Ester, dessen Carbonsäurerest sich von C₅- bis C₁₆-Säuren ableitet und dessen Hydroxylrest sich von Monomeren, Dimeren oder Trimeren der Milchsäure oder eines ihrer Salze oder einem Polyglycerin aus 2 bis 10 Molekülen Glycerin ableitet, wobei pro mol Polyglycerin 1 bis 3 mol Carbonsäure vorliegen.

2. Topische Zusammensetzung nach Anspruch 1, **dadurch gekennzeichnet, daß** die Komponente (a) in der Zusammensetzung in einer Menge von 0,02 bis 2 Gew.% vorliegt.

3. Topische Zusammensetzung nach Anspruch 1 oder 2, **dadurch gekennzeichnet, daß** die Komponente (b) aus der Gruppe, bestehend aus Polyglycerin-10-tricaprylat, Polyglycerin-10-trilaurat, Polyglycerin-2-oleat, Natriumlauroyllactylat, Natriumcocoyllactylat, Caprin/Caprylsäuretriglycerid und Mischungen davon, ausgewählt wird.

4. Topische Zusammensetzung nach einem der Ansprüche 1 bis 3, **dadurch gekennzeichnet, daß** die Komponente (b) in der Zusammensetzung in einer Menge von 0,5 bis 30 Gew.% vorliegt.

5. Topische Zusammensetzung nach einem der Ansprüche 1 bis 4, **dadurch gekennzeichnet, daß** die Zusammensetzung weiterhin (c) mindestens einen Coemulgator, ausgewählt aus der Gruppe, bestehend aus Glycerin- und Sorbitanesterderivaten, Cetearylalkohol, Cetearylesterderivaten und Mischungen davon, enthält.

6. Topische Zusammensetzung nach Anspruch 5, **dadurch gekennzeichnet, daß** die Komponente (c) in der Zusammensetzung in einer Menge von 0,1 bis 40 Gew.% vorliegt.

7. Topische Zusammensetzung nach einem der Ansprüche 1 bis 6, **dadurch gekennzeichnet, daß** die Zusammensetzung weiterhin (d) mindestens ein lipophiles Lösungsmittel enthält.

8. Topische Zusammensetzung nach Anspruch 7, **dadurch gekennzeichnet, daß** die Komponente (d) ausgewählt wird aus der Gruppe, bestehend aus Ethylbutylacetylaminopropionat, Ethanol, Isopropanol, Isopropylmyristat und Mischungen davon.

9. Topische Zusammensetzung nach Anspruch 7 oder 8, **dadurch gekennzeichnet, daß** die Komponente (d) in der Zusammensetzung in einer Menge von 0,1 bis 20 Gew.% vorliegt.

10. Topische Zusammensetzung nach einem der Ansprüche 1 bis 9, **dadurch gekennzeichnet, daß** die Zusammensetzung weiterhin (e) mindestens einen Hilfsstoff, ausgewählt aus Antioxidantien und UV-Filtern, enthält.

11. Verfahren zur Herstellung der topischen Zusammensetzung nach einem der Ansprüche 1 bis 10, umfassend die Schritte
- Herstellung einer Phase A durch Mischen der Komponenten (a) und (b) und
- Einarbeitung der erhaltenen Phase A in eine Phase B, enthaltend einen Träger.

12. Verfahren nach Anspruch 11, **dadurch gekennzeichnet, daß** der Träger deionisiertes Wasser sowie Mischungen aus deionisiertem Wasser und Alkoholen umfaßt.

13. Verfahren nach Anspruch 11 oder 12, **dadurch gekennzeichnet, daß** Phase A und/oder Phase B weiterhin mindestens eine der Komponenten (c) bis (d) enthält.

14. Verfahren nach einem der Ansprüche 11 bis 13, **dadurch gekennzeichnet, daß** Phase A unter Rühren bei 60 bis 100°C hergestellt wird.

15. Verfahren nach einem der Ansprüche 11 bis 14, **dadurch gekennzeichnet, daß** die Phasen A und/oder B vor dem Einarbeitungsschritt auf 60 bis 100°C erwärmt werden.

16. Verwendung der topischen Zusammensetzung nach einem der Ansprüche 1 bis 10 zur Herstellung eines Medikaments zur Prophylaxe und/oder Behandlung von Hauterkrankungen und/oder Entzündungsreaktionen der Haut.

17. Verwendung der topischen Zusammensetzung nach einem der Ansprüche 1 bis 10 zur kosmetischen Pflege der Haut.

## Claims

1. A topical composition, comprising
(a) at least one aryl oxime of the Formula (I) and
(b) at least one emulsifier, 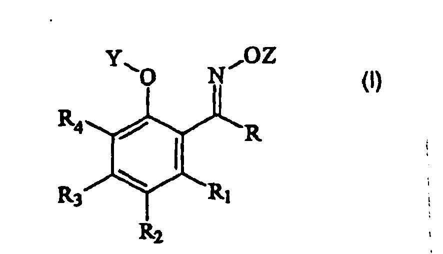 wherein:
Y, Z represent independently from each other H, C₁₋₁₈ alkyl, C₂₋₁₈ alkenyl, C₂₋₁₈ carboxy alkyl, C₃₋₁₈ carboxy alkenyl or C₂₋₁₈ alkanoyl;
R represents C₁₋₁₈ alkyl, C₂₋₁₈ alkenyl, C₃₋₈ cycloalkyl, aryl, aralkyl, heteroaryl, heteroaralkyl or condensed systems;
R₁, R₂, R₃ and R₄ represent independently from each other H, C₁₋₁₂ alkyl, C₂₋₁₂ alkenyl, C₁₋₁₂ alkoxy, C₃₋₈ cycloalkoxy, aryl, aryloxy, aralkyl, heteroaryl, heteroaralkyl, carboxy, hydroxy, chlorine, dialkyl amine or sulfonyl,
**characterized in that** the component (b) is selected from the group consisting of at least one ester, the carboxylic acid residue of which is derived from C₅-C₁₆ acids and the hydroxyl residue of which is derived from monomers, dimers or trimers of lactic acid or one of its salts or a polyglycerin of 2 to 10 molecules of glycerin whereby 1 to 3 moles of carboxylic acid are present per mole of polyglycerin.

2. Topical composition according to claim 1, **characterized in that** the component (a) is present in the composition in an amount of 0.02 to 2 wt. %.

3. Topical composition according to claim 1 or 2, **characterized in that** the component (b) is selected from the group consisting of polyglycerin 10-tricaprylate, polyglycerin-10-trilaurate, polyglycerin-2-oleate, sodium lauroyl lactate, sodium cocoyl lactate, capric/caprylic acid triglyceride and mixtures thereof.

4. Topical composition according to any of claims 1 to 3 **characterized in that** the component (b) is present in the composition in an amount of 0.5 to 30 wt.%.

5. Topical composition according to any of claims 1 to 4, **characterized in that** the composition further comprises (c) at least one co-emulsifier selected from the group consisting of glycerin and sorbitan ester derivatives, cetearyl alcohol, cetearyl ester derivatives and mixtures thereof.

6. Topical composition according to claim 5, **characterized in that** the component (c) is present in the composition in an amount of 0.1 to 40 wt.%.

7. Topical composition according to any of claims 1 to 6, **characterized in that** the composition further comprises (d) at least one lipophilic solvent.

8. Topical composition according to claim 7, **characterized in that** the component (d) is selected from the group consisting of ethyl butyl acetyl amino propionate, ethanol, isopropanol, isopropyl myristate and mixtures thereof.

9. Topical composition according to claims 7 or 8, **characterized in that** the component (d) is present in the composition in an amount of 0.1 to 20 wt.%.

10. Topical composition according to any of claims 1 to 9, **characterized in that** the composition further comprises (e) at least one auxiliary, selected from antioxidants and UV filters.

11. Process for the preparation of a topical composition according to any of claims 1 to 10, comprising the following steps
- preparation of a phase A by mixing the components (a) and (b), and
- incorporation of the obtained phase A into a phase B, containing a carrier.

12. Process according to claim 11, **characterized in that** the carrier comprises de-ionized water, as well as mixtures of de-ionized water and alcohols.

13. Process according to claims 11 or 12, **characterized in that** phase A and/or phase B further contain at least one of the components (c) to (d).

14. Process according to any of claims 11 to 13, **characterized in that** phase A is prepared at 60 to 100°C with stirring.

15. Process according to any of claims 11 to 14, **characterized in that** the phases A and/or B are heated to 60 to 100°C prior to the incorporation step.

16. Use of the topical composition according to any of claims 1 to 10 for the manufacture of a medicament for prophylaxis and/or treatment of skin diseases and/or inflammation responses of the skin.

17. Use of the topical composition according to any of claims 1 to 10 for the cosmetical care of the skin.

## Revendications

1. Composition topique contenant
(a) au moins une aryloxime de formule (I) et
(b) au moins un émulsifiant 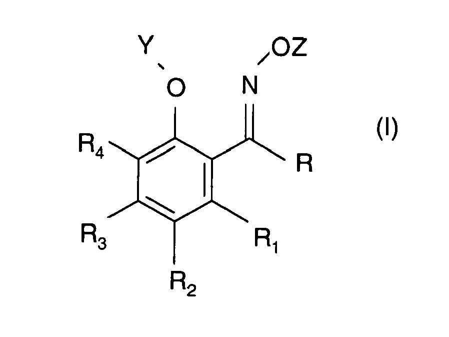 où :
Y, Z signifient indépendamment l'un de l'autre H, C₁₋₁₈-alkyle, C₂₋₁₈₋alcényle, C₂₋₁₈-carboxyalkyle, C₃₋₁₈-carboxyalcényle ou C₂₋₁₈-alcanoyle ;
R signifie C₁₋₁₈-alkyle, C₂₋₁₈-alcényle, C₃₋₈-cycloalkyle, aryle, aralkyle, hétéroaryle, hétéroaralkyle ou des systèmes condensés ;
R₁, R₂, R₃, R₄ signifient indépendamment les uns des autres H, C₁₋₁₂-alkyle, C₂₋₁₂-alcényle, C₁₋₁₂-alcoxy, C₃₋₈-cycloalcoxy, aryle, aryloxy, aralkyle, hétéroaryle, hétéroaralkyle, carboxy, hydroxy, le chlore, dialkylamine ou sulfonyle,
**caractérisée en ce que** le composant (b) est choisi dans le groupe consistant en au moins un ester dont le groupement acide carboxylique dérive d'acides en C₅ à C₁₆ et dont le groupement hydroxyle dérive de monomères, de dimères ou de trimères de l'acide lactique ou de l'un de ses sels ou d'une polyglycérine constituée par 2 à 10 molécules de glycérine, où 1 à 3 moles d'acide carboxylique sont présentes par mole de polyglycérine.

2. Composition topique selon la revendication 1 **caractérisée en ce que** le composant (a) est présent dans la composition en une quantité de 0,02 à 2 % en masse.

3. Composition topique selon la revendication 1 ou 2
**caractérisée en ce que** le composant (b) est choisi dans le groupe consistant en le polyglycérine-10-tricaprylate, le polyglycérine-10-trilaurate, le polyglycérine-2-oléate, le lauroyllactylate de sodium, le cocoyllactylate de sodium, un triglycéride d'acide caprique/caprylique et leurs mélanges.

4. Composition topique selon l'une des revendications 1 à 3 **caractérisée en ce que** le composant (b) est présent dans la composition en une quantité de 0,5 à 30 % en masse.

5. Composition topique selon l'une des revendications 1 à 4 **caractérisée en ce que** la composition contient en outre (c) au moins un co-émulsifiant, choisi dans le groupe consistant en les dérivés d'esters de glycérine et de sorbitan, l'alcool cétéarylique, les dérivés d'esters cétéaryliques et leurs mélanges.

6. Composition topique selon la revendication 5 **caractérisée en ce que** le composant (c) est présent dans la composition en une quantité de 0,1 à 40 % en masse.

7. Composition topique selon l'une des revendications 1 à 6 **caractérisée en ce que** la composition contient en outre (d) au moins un solvant lipophile.

8. Composition topique selon la revendication 7 **caractérisée en ce que** le composant (d) est choisi dans le groupe consistant en le butylacétylaminopropionate d'éthyle, l'éthanol, l'isopropanol, le myristate d'isopropyle et leurs mélanges.

9. Composition topique selon la revendication 7 ou 8 **caractérisée en ce que** le composant (d) est présent dans la composition en une quantité de 0,1 à 20 % en masse.

10. Composition topique selon l'une des revendications 1 à 9 **caractérisée en ce que** la composition contient en outre (e) au moins un adjuvant choisi parmi les antioxydants et les filtres UV.

11. Procédé de production de la composition topique selon l'une des revendications 1 à 10 comprenant les étapes
- production d'une phase A par mélange des composants (a) et (b) et
- incorporation de la phase A obtenue dans une phase B contenant un support.

12. Procédé selon la revendication 11 **caractérisé en ce que** le support comprend de l'eau désionisée ainsi que des mélanges d'eau désionisée et d'alcools.

13. Procédé selon la revendication 11 ou 12 **caractérisé en ce que** la phase A et/ou la phase B contiennent en outre au moins l'un des composants (c) à (d).

14. Procédé selon l'une des revendications 11 à 13 **caractérisé en ce que** la phase A est produite sous agitation à 60 à 100°C.

15. Procédé selon l'une des revendications 11 à 14 **caractérisé en ce que** les phases A et/ou B sont chauffées à 60 à 100°C avant l'étape d'incorporation.

16. Utilisation de la composition topique selon l'une des revendications 1 à 10 pour la production d'un médicament pour la prophylaxie et/ou le traitement des maladies de la peau et/ou des réactions inflammatoires de la peau.

17. Utilisation de la composition topique selon l'une des revendications 1 à 10 pour les soins cosmétiques de la peau.
